# EUROPEAN PATENT SPECIFICATION

(11) **EP 0 210 039 B1**
(45) Date of publication and mention of the grant of the patent: **20.01.1993**
(21) Application number: 86305431.8
(22) Date of filing: 15.07.1986
(51) Int. Cl.: A61K 9/22, A61K 37/36

(54) **Controlled release implants for administration of recombinant growth hormones**
Implantate mit verzögerter Abgabe für die Anwendung von rekombinanten Wachstumshormonen
Implants à effet retardé pour administration d'hormones de croissance recombinées

(30) Priority: 15.07.1985 US 755093
(43) Date of publication of application: 28.01.1987
(73) Proprietor: IMCERA Group Inc., Northbrook, IL 60062 (US)
(72) Inventor: Janski, Alvin M., Northbrook Illinois 60062 (US); Yang, Ren-Der, Terre Haute Indiana 47803 (US); SIVARAMAKRISHNAN, Kallidaikurichi N, Terre Haute Indiana 47802 (US)
(74) Representative: Holmes, Michael John

(56) References cited:
- EP-A- 0 085 036
- EP-A- 0 161 640
- EP-A- 0 201 219
- EP-A- 0 204 476

## Description

The present invention relates to improved controlled release implants for the subcutaneous administration of bioactive recombinant growth hormones (i.e. growth hormones produced by recombinant DNA technology) at a controlled and continuous rate to an animal host. The invention further relates to a method of purifying a protein having the activity of an animal growth hormone and which has been produced by recombinant DNA technology, e.g. recombinant bovine growth hormone, or porcine growth hormone in a form suitable for use in controlled release implants.

Growth hormones are proteins that are involved in regulating protein metabolism as well as fat, carbohydrate, and mineral metabolism. Growth hormones affect the metabolic processes of the body by increasing the rate of cellular protein synthesis and decreasing protein degradation, as well as by increasing the rate of use of fatty acids and decreasing the rate of use of carbohydrates for production of energy in the body.

Bovine growth hormone (BGH) and porcine growth hormone (PGH) are proteins containing 191 amino acid residues. These proteins are synthesized in the anterior pituitary gland as "pre-growth hormones" having 26 additional amino acid residues attached at the amino terminal end. These 26-amino acid residue sequences are cleaved off prior to secretion from the pituitary cells, yielding the mature hormones. Field trials using BGH purified from pituitary glands demonstrated increased milk production and improved feed-to-milk conversion in cows to which the hormone was administered (Machlin, L.J., Journal of Diary Science, 56:575-580 [1973]). The potential economic value of this hormone sparked interest in obtaining BGH in commercial quantities at reasonable cost. Field trials of native PGH have shown increased growth rates in young swine receiving the hormone.

Thus, much work in recent years has focused on obtaining microbial synthesis of these commercially valuable hormones using recombinant DNA technology. Gene cloning and manipulation techniques, well known in the art, have been used to produce recombinant expression vectors capable of directing the synthesis of BGH and PGH. For example, microorganisms transformed with BGH-encoding cDNA linked to a regulatory system have been shown to produce the desired hormone. Keshet et al., (Nucleic Acids Research, 9:19-30 [1981]) reported the cloning and low level expression in E. coli of a full length BGH polypeptide as a fusion protein with a portion of pBR322-encoded β-lactamase. In European Patent Application Publication No. 0 103 395, construction of several expression vectors, including vectors encoding BGH polypeptides with varying portions of the amino-terminal end deleted, is described. BGH polypeptides with varying portions of the amino-terminal end of the mature hormone deleted were found to retain biological activity and to be expressed at much higher levels than was the complete hormone in the expression systems described.

Administration of BGH to cattle and PGH to swine has hitherto been only marginally successful. Methods of delivery of drugs that are well known in the art include oral, nasal, rectal, topical, and parenteral injection routes of administration. However, it is inconvenient to administer drugs to cattle and swine by these methods because of the large expense and amount of time required to deliver the drug to each member of a large group of animals on a daily basis.

Subcutaneous implants provide an alternative means for administering sustained, effective dosages of recombinant BGH and PGH to each animal. The implant contains a hormone reservoir surrounded by a protective wall permeable to the hormone. The advantage of these delivery systems is that they provide for controlled and predictable release rates of the hormones to the animals over an extended period of time. Unfortunately, we have found that contolled release devices containing proteins having BGH or PGH activity produced by transformant microorganisms in fermentation media are subject to swelling and partial disintegration after implantation. This phenomenon dilutes the hormone in the implant and adversely affects the rate of release of the hormone. Therefore, there is a commercial need for improved controlled release implants for the subcutaneous administration of recombinant proteins having growth hormone activity to animals.

Unlike a native animal growth hormone, a recombinant protein having the activity of an animal growth hormone which has been purified by conventional techniques contains a substantial amount of salt which is present largely as a result of salts in buffers used in the recovery operations. The present invention is based on our discovery that reduction of the salt level of a recombinant protein having growth hormone activity to less than 5% by weight prior to incorporation into a controlled release implant eliminates the swelling problem previously encountered when purified recombinant proteins having growth hormone activity were used for this purpose.

According to one aspect of the present invention, we provide a controlled release implant for subcutaneous administration to an animal of a growth promoting amount of a recombinant protein havng growth hormone activity, characterised in that said protein is present in a form having a salt content of less than 5% by weight based on the weight of said protein. Preferred controlled release implants according to the present invention have a compressed matrix comprising the recombinant protein mixed with a biocompatible water-insoluble polymer.

In a second aspect, the present invention provides a method of preparing such an implant which comprises:
a) subjecting a solution of a recombinant protein having the activity of an animal growth hormone, which has been recovered from transformant cells in a fermentation medium, to one or more dialysis steps at a pH no lower than physiological pH so as to obtain an aqueous solution of said protein having a reduced salt content relative to that of the starting solution;
b) lyophilizing an aqueous solution of said protein at a pH no lower than physiological pH to produce a lyophilisate which has a salt content of less than 5% by weight based on the weight of said recombinant protein; and
c) formulating the lyophilized protein thus obtained into said implant.

In step (c), initially a compressible composition may be formed comprising the lyophilized low salt recombinant protein mixed with a biocompatible water-insoluble polymer, which is then compressed to give a unitary dosage matix for the implant.

By means of a method as hereinbefore described for preparation of an implant, an implant may be obtained wherein a recombinant protein having growth hormone activity is in the presence of buffer salts at less than 5% by weight based on the weight of recombinant protein which will result in the pH within the implant being about physiological pH (i.e. about pH 7.4) upon wetting in a physiological environment. An implant according to the present invention which upon subcutaneous implantation sets up little or no pH gradient between its interior and its in vivo environment is highly desirable since a more predictable and steady rate of protein release in vivo is ensured.

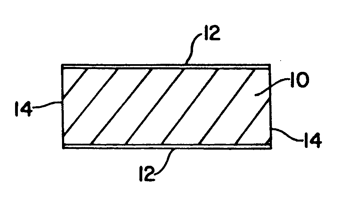

### BRIEF DESCRIPTION OF THE DRAWING

The single figure is a cross-sectional representation of a cylindrical implant for the controlled-release administration of a recombinant protein having growth hormone activity to an animal.

### DETAILED DESCRIPTION OF THE INVENTION

We have developed improved implants for controlled release into an animal of recombinant proteins having growth hormone activity, which implants contain such recombinant proteins in lyophilised form having been treated to lower the salt content to below 5% by weight.

Hereinafter, controlled release implants according to the present invention wherein the recombinant protein has bovine growth hormone or porcine growth hormone activity and methods for preparing such implants will be described in greater detail. However, it should be understood that any recombinant protein having the activity of an animal growth hormone may be employed in the construction of an implant within the scope of the invention after appropriate purification. As used hereinafter, unless the context otherwise indicates, the terms "bovine growth hormone" and "BGH" should be understood to refer to any protein having bovine growth hormone activity and the terms "porcine growth hormone" and "PGH" should similarly be understood to refer any protein having porcine growth hormone activity. These terms thus include bioactive fragments of the mature hormones which may, for example, have varying portions of the amino terminal ends of the hormones deleted, and bioactive analogs with one or more substitutions and/or modifications in the BGH and PGH sequences which do not destroy the biological activity of the polypeptides. BGH and PGH polypeptides lacking various portions of the amino terminal end of the mature hormones have been shown to retain biological activity.

The cloning and microbial expression of BGH and PGH genes can be carried out using conventional techniques of molecular biology. The plasmids that direct the expression of BGH and PGH in transformed microorganisms can be any suitable growth hormone-encoding plasmids. The host microorganisms may be either Gram-positive or Gram-negative. Gram-negative microorganisms include those selected from the genus Esherichia. Gram-positive microorganisms include those selected from the genus Bacillus and the genus Streptomyces. The precise host microorganism employed is not critical.

In producing BGH for use in the preparation of an implant of the present invention, we employed an E. coli host strain HB101 or MC1061 transformed with a first plasmid, pL-mu-Δ9C143, coding for mature bovine growth hormone less its nine N-terminal amino acids and a serine residue at the N-terminal end, under the control of a phage lambda promoter and a second plasmid, pcI857, which codes for the temperature sensitive λcI857 repressor protein. Construction of a transformant strain of this type is described in detail in European Patent Application Publication No. 0 103 395. The HB101 transformant strain, identified as E. coli IMC No. 1, has been deposited at the American Type Culture Collection, Rockville, Maryland under accession no. ATCC 53030. It will be readily apparent, however, that a process of this invention may equally employ recombinant BGH produced by any host/vector combination.

For the production of PGH, we employed an E. coli host strain HB101 transformed with the plasmid pcI857 and the plasmid pL-mu-Δ7 SGH coding for porcine growth hormone less its seven N-terminal amino acids under the control of a phage lambda promoter. This transformant strain, identified as E. coli IMC No. 2, has been deposited at the American Type Culture Collection, Rockville, Maryland under accession no. 53031.

Following the expression in microbial culture of a cloned recombinant BGH or PGH gene, the recombinant protein with growth hormone activity may be recovered using various techniques of preliminary fractionation. When a sufficient amount of the recombinant BGH or PGH has been expressed by the transformed microorganisms, the cells are separated from the bulk of the culture medium, normally by centrifugation. The expressed protein is then obtained from the cells, in the case of a non-secreting host such as E. coli, or from the medium, in the case of a secreting host such as B. subtilis. In the case of a non-secreting host such as E. coli, the cells must be disrupted in order to release the protein. Disruption can be achieved mechanically using, for example, a French press or a Manton-Gaulin homogenizer, and the cell debris removed, or it can be achieved chemically. The resulting composition which is subjected to further purification may contain, in addition to the microbially produced protein, other proteins which are expressed by the transformant host microorganism, e.g. residual structural proteins of the host microorganism, microbial metabolites including endotoxins, residual constituents of the fermentation medium and any other residual materials resulting from fermentation and expression. Upon completion of the cell disruption, the BGH or PGH may then be separated from the cell debris and the impurities by methods such as centrifugation, large scale chromatography and batch extraction techniques.

As previously indicated, subsequent purification to prepare recombinant BGH or PGH for use in an implant of the present invention may be achieved by a procedure involving dialysis of the recombinant BGH or PGH followed by lyophilization. As used herein, the term "dialysis" refers to any technique in which salt is removed from a recombinant protein solution by selective transport of salt ions across a semi-permeable membrane or surface boundary with retention of the desired protein molecules on the other side of the membrane or surface boundary. Any of the known methods of dialysis may be used with a variety of types of equipment. For example, small molecules in a protein solution may be dialyzed or ultrafiltered using hollow fiber ultrafiltration systems. In this procedure, dialysis buffer solution of low ionic strength is passed through bundles of semi-permeable hollow fibers. Small molecules in the protein solution that surrounds the fibers are capable of passing through the membranous fiber wall so as to reduce the ionic strength of the protein solution. As used herein, the term "dialysis" also includes techniques such as size exclusion chromotography, wherein the salt ions are able to cross the surface boundary of a particulate medium while the desired protein remains in the exudate or eluate.

A convenient dialysis technique for small scale dialysis of a recovered recombinant protein with growth hormone activity involves adding the recombinant protein preparation to a buffer solution and placing this mixture into a sac made by knotting each end of a piece of semi-permeable dialysis tubing. The sealed tubing containing the recombinant protein is dialysed against increasingly lower concentrations of buffer until the recombinant protein is at least 95% salt free. The buffer preferably does not contain sodium chloride. The pH of the dialysis buffer solution in the case of recombinant BGH is maintained within the range of 9.6 to 10.0, preferably 9.8. The temperature is generally maintained within the range of 5°C to 15°C. A particularly useful buffer for further purification of recombinant BGH is a sodium bicarbonate/sodium carbonate buffer of the composition 25mM NaHCO₃, 21 mM Na₂CO₃. This buffer has been called "Cornell Buffer minus sodium chloride" and it is designated by the symbol CB⁻. The BGH may, for example, be dialyzed directly from concentrated buffer into dilute buffer, e.g. a 0.01x or 0.02x dilution of CB⁻buffer, or may be dialyzed by stepwise dialysis against progressive dilutions of CB⁻buffer, e.g. against 1x, 0.20x, 0.05x and 0.01x dilutions of CB⁻ buffer. The one or more dialysis steps may, for example, be preceded and/or followed by one or more ultrafiltration steps to concentrate the recombinant protein. These procedures are effective for removing salt and lowering the ionic strength.

In a procedure which is useful for removing salt from recombinant BGH on a larger scale, a purified dilute solution of recombinant BGH (generally less than 1.0 mg/ml) is concentrated to greater than 1.0 mg/ml in a cross-flow membrane filtration unit (such as an Amicon DC-10 unit) using a membrane that will pass most of those molecules having a molecular weight of less than 10,000. The resulting concentrated product solution in 60 mM ethanolamine buffer at pH 9.0 is diafiltered using the same membrane unit against 2 volumes of 50% strength CB⁻ buffer and then 5 volumes of 2% strength CB⁻buffer. The resulting product retentate, now in 2% CB⁻buffer, is then further concentrated to a final product concentration of 5-20 mg/ml. Before lyophilization, the final concentrate is clarified by centrifugation followed by 0.2µm(µ) microporous filtration.

The procedures described above generally achieve a recovery efficiency between 60 to 80% with the resulting lyophilized product containing less than 5% by weight salts (pH 9.8).

In the lyophilization step, a solution of low salt recombinant BGH may be placed in shallow trays that are then put on the shelves in a high- vacuum chamber. The shelves are maintained by refrigeration at a temperature of about -40°C during freezing. During sublimation of water substance, the shelf temperature is maintained at approximately 25°C.

After each step of the purification procedure, identification of the recombinant protein product may be confirmed by any suitable means. A convenient procedure involves resolubilization of the product followed by Bio-Rad protein assay and radio-receptor assay.

The salt content of recombinant porcine growth hormone may be reduced by the foregoing methods used for BGH. However, a preferred method for reduction of the salt content of recombinant PGH entails dialyzing the PGH preparation against a buffer having a physiological pH of about 7.4.
This buffer has the following composition:
2-5 mM Tris
pH ≃ 7.4 adjusted with HC1
Tris = Tris (hydroxymethyl) amino-methane
In an especially preferred procedure for reducing the salt content of recombinant PGH, a purified dilute solution of the recombinant protein (generally less than 1.0 mg/ml) is concentrated to greater than 1.0 mg/ml in a cross-flow membrane filtration unit (such as an Amicon DC-10 unit) using a membrane that will pass most molecules having a molecular weight of less than 10,000. The resulting concentrated product solution in 60 mM ethanolamine buffer at pH 9.0 is diafiltered using the same membrane unit against 5 volumes of Tris buffer at 2-5 mM concentration and about pH 7.4.

The resulting product retentate, now in 2- 5 mM Tris buffer, is then further concentrated to a final concentration of between 5 and 20 mg/ml. Before lyophilization, the final concentrate is clarified by centrifugation followed by 0.2µm(µ) micro-porous filtration.

The procedures described above for further purification of recombinant PGH produce a final lyophilized product with less than 5% by weight of salts. The recovery yield is comparable to CB⁻buffer finishing for PGH recombinant, but lower than for recombinant BGH when treated as hereinbefore described.

Either lyophilized recombinant BGH or PGH with a salt content of less than 5% by weight may be incorporated in an implant for subcutaneous administration as described in the following paragraphs.

For the controlled administration of a recombinant protein having growth hormone activity (recombinant GH) from solid implants, it is advantageous to have a matrix consisting of the GH, a polymer as a filler and other suitable additives. It is important that the polymeric filler be biocompatible and compatible with the GH.

For example, if the polymer is too hydrophobic, it may bind the GH so strongly that the protein may not be released readily. In extreme cases, the GH may even be denatured by the hydrophobic matrix and thus rendered inactive. On the other hand, if the polymer is too hydrophilic, penetration of water into the implant can be rapid. The wet implant may facilitate aggregation of the GH which can result in decreased solubility and/or bioactivity. Thus, the ideal polymeric filler should exhibit a balance between the hydrophobic and hydrophilic forces.

Ethyl cellulose (EC) is a commercially available, water-insoluble polymer which fits the requirements of a polymeric filler for an implant containing GH. It is a derivative of cellulose in which the hydroxyl groups have been partially etherified. The ether groups provide the hydrophobicity while the hydroxyl groups give hydrophilicity to the polymer. By altering the degree of etherification, one can achieve the desired balance between the two types of interaction. Another advantage of EC is the presence of the unsubstituted hydroxyl groups which may stabilize the GH in the wet implant and minimize aggregation of the protein. A third advantage is the ability of EC to act as binder in tablet preparations. By controlling the amount of the EC in the matrix, it is possible to control the compactness of the solid pellet. This can be used to control the water penetration into the implant and the disintegration of the pellet.

A recombinant GH, being a delicate protein, may easily be denatured when brought into contact with organic solvents. In conventional tablet formulations, the drug is usually mixed with a solution of the polymeric filler, dried and granulated. This may not be desirable for the formulation of a GH as a solid implant. EC offers another advantage in that it can be formulated in the dry state with the GH, thus avoiding the potentially damaging exposure to organic solvents.

In summary, EC can be very useful in the formulation of a solid implant of the present invention. The amount of EC can vary from 10 to 50% depending on the type of release profile needed. It can also be used in conjunction with other suitable additives such as sucrose, lactose, magnesium stearate, etc. which are employed in conventional tablet formulation for various purposes. A matrix for an implant of the present invention may, for example, contain 30% by weight of a lyophilized recombinant GH (salt content less than 5% by weight), 30% by weight of EC and 40% by weight of sucrose.

Referring to the single Figure, a typical controlled release implant of the present invention incorporating recombinant BGH can be produced as follows. Lyophilized recombinant BGH having a salt content of less than 5% by weight (25 parts; particle size: 150-250 micrometres (microns)) and EC (25 parts; particle size 150-250 micrometres (microns)) are mixed in a vial using a vortex shaker. The matrix is then pelleted with a Stoke's machine to give generally cylindrical shaped pellets containing a growth promoting amount of the recombinant protein e.g. pellets weighing 50 mg and measuring 4.0 mm in diameter and 3.9 mm in length. The pellets are placed in microporous polyethylene (MPE) tubes and the ends of the tubes sealed with non-porous polyethylene film. The resultant implant of generally cylindrical shape for the controlled release of recombinant BGH is illustrated in cross-section in the single Figure. The implant contains a central core pellet which is surrounded along its length by a releasing surface 12 of the microporous polyethylene film. At the end of the cylinder are non-releasing surfaces 14 of non porous polyethylene. Upon subcutaneous implantation in cattle, the releasing surface 12 of MPE acts as a barrier to slow the rate of diffusion of recombinant BGH out of the implant, thereby resulting in prolonged release of the protein.

Similar implants within the scope of the present invention may, of course, be constructed using an alternative lyophilized recombinant GH having a suitably low salt content. If desired, other microporous polymer films may be used in place of MPE. These include, for example, microporous films of ethyl cellulose, polycaprolactone and polymethyl methacrylate. The non-releasing surface 14 of non-porous polyethylene (or other non-porous polymer) serves to prevent the recombinant GH from being released through the ends of the implant.

The following examples serve to further illustrate this invention.

### Example 1

Δ 9(Ser)BGH (ie recombinant BGH corresponding in amino acid sequence to amino acid residues 10 to 191 of mature BGH with a serine residue at the N-terminus) was obtained by lysis of E. coli (MC1061) transformant cells which had been grown in a fermentation medium at 28°C until the A₅₅₀ of the medium reach 50-60 and then induced to express BGH by raising the temperature to 42°C. After removal of unwanted cellular material and recovery by conventional techniques of protein solubilization and purification, there was obtained 1500 ml of aqueous solution of Δ9 BGH at a concentration of 110 ppm. This solution was concentrated to 350 ml by ultrafiltration across a membrane which passed molecules below 5,000 molecular weight. The solution was clarified by centrifugation and the supernatant was concentrated to 32 ml by ultrafiltration. The solution was then dialyzed by sack dialysis against approximately 320 ml of CB⁻ buffer (25 mM NaHCO₃, 21 mM Na₂CO₃, pH 9.8). The solution was concentrated to 6.2 ml by ultrafiltration across a membrane which passed molecules below 10,000 molecular weight. The solution was clarified by centrifugation and filtration through a 0.2µm(µ) microporous filter to give 6.2 ml having a protein concentration of 11.4 mg/ml. The preparation was diluted to about 2 mg/ml in 1X CB⁻ and a 10 ml sample was dialyzed overnight in a Spectropore 1 (6,000 - 8,000 Dalton cutoff) tubing against 1,600 ml 1X CB⁻, pH 9.67. The product was further dialyzed to remove salt as follows:
A one milliliter sample of the product was stepwise dialyzed against 0.2X CB⁻ for 4 hours, then 0.05X CB⁻ for 4 hours, and finally 0.01X CB⁻ overnight. Another one milliliter sample of the product was directly dialyzed against 0.01X CB⁻. After dialysis, each bag was mixed by inversion.

One ml aliquots were removed from each dialysis bag and transferred to separate silanated glass liquid-scintillation vials. The samples were shell-forzen in dry ice-acetone and lyophilized overnight at about 7 micrometres (microns) Hg pressure.

After each dialysis, and following lyophilization, samples of the product were solubilized in phosphate buffered saline (10 mM Na₂HPO₄, 10 mM NaH₂PO₄, 140 mM NaC1, pH 7.4), and the purity of the product was determined by Bio-Rad protein assay and radio receptor assay. The radio receptor assay, as described by J. Roth, Methods in Enzymology, 37 (1975), 66-81 (Chapter 4), is a competitive binding assay in which the test sample containing BGH and a sample of known concentration of ¹²⁵I-labelled BGH are incubated with a suspension of small particulate vesicles of pregnant rabbit liver membrane growth hormone receptors. Bound label is separated from unbound label by centrifugation and the centrifugation pellet containing the bound label is placed in a gamma counter. The BGH titer in the sample was determined by comparison with a standard curve. The results of solubility and radio receptor assay experiments are presented in Table 1 for the samples were dialyzed directly or in stepwise fashion as well as for samples which did not undergo dialysis. Dialysis, lyophilization, and resolubilization in phosphate buffered saline of Δ9 (Ser) BGH led to retention of over 92% of the hormone's solubility, 95% recovery of the hormone, and retention of radio receptor binding activity.

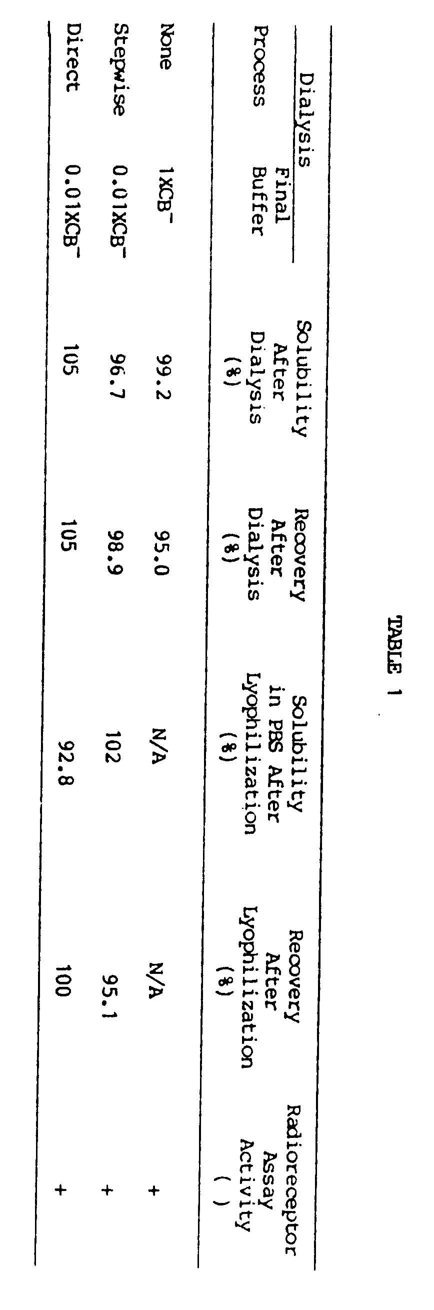

### Example 2

One hundred liters of purified Δ9 (Ser) BGH at a concentration of approximately 150 mg/L in 60 MM ethanolamine buffer was obtained from E. coli IMC No. 1 cells (ATCC 53030) which had been grown in a fermentation medium. The solution was concentrated to about 2100 mg/L in a cross-flow membrane filtration unit (e.g., Amicon in a cross-flow membrane filtration unit (e.g., Amicon (DC-10) equipped with a membrane that passed most of those molecules with molecular weights below 10,000. The resulting concentrated product solution in the 60 mM ethanolamine buffer at pH 9.0 was diafiltered against a 2X volume of 50% strength CB⁻ buffer and then 5 volumes of 2% CB⁻ buffer. The resulting retentate, now in 2% CB⁻ buffer, was then further concentrated in an Amicon DC-10 unit to a final value of 14 mg/mL. Before lyophilization, the final concentrate is clarified by centrifugation and filtration through a 0.2 micrometre (micron) microporous filter.

The filter solution was lyophilized to give a product that contains less then 5% by wt. salts and gives a solution with a pH of 9.8 when added to deionized water.

The procedure desribed above gave a yield of about 90%, however, more typical yields are 60-80%.

### Example 3

Sixty-three liters of purified Δ7 PGH at a concentration of approximately 80 mg/L in 60 mM ethanolamine buffer was obtained from E. coli IMC No. 2 cells (ATCC 53031) which had been grown in a fermentation medium. The solution was concentrated to about 800 mg/L in a cross-flow membrane filtration unit (e.g., Amicon DC-10) equipped with a membrane that passes most of those molecules with molecular weights below 10,000. The resulting concentrated product solution in the 60 mM ethanolamine buffer at pH 9.0 was diafiltered against a 5X volume of Tris HCL buffer at 2-5 mM concentration and a pH of 7.4. The resulting product retentate, now in 2-5 mM Tris buffer, was then further concentrated in an Amicon DC-10 unit to a final value of from 2.5 to 20 mg/mL. Before lyophilization, the final concentrate is clarified by centrifugation and filtration through a 0.2 micrometre (micron) microporous filter.

The filtered solution was lyophilized to give a product that contains less than 5% by wt. salts and gives a solution with a pH of 7.4 when added to deionized water.

When an implant of PGH in the presence of pH 7.4 buffer salts is wetted by body fluids at about pH 7.4, little or not pH gradient should exist, allowing for a more predictable release rate of PGH from the implant.

The procedure described above gave a yield of at least 60%.

### Example 4

A formulation for the preparation of growth hormone implants is prepared from the following ingredients:

| Ingredient | Parts by Weight |
|---|---|
| BGH or PGH | 30 |
| Sucrose | 40 |
| Ethyl cellulose | 30 |

The ingredients are mixed in a vial using a vortex shaker. The mixture is pelleted using a Stoke's machine to give cylindrical pellets weighing 50 mg and measuring 4.0 mm in diameter and 3.9 mm in length. The pellets are placed in microporous polyethylene tubes and the ends are sealed with non-porous polyethylene.

## Claims (Claims for the following Contracting State(s): BE, CH, DE, FR, GB, IT, LI, LU, NL, SE)

1. A controlled release implant for subcutaneous administration to an animal of a growth promoting amount of a recombinant protein having growth hormone activity, characterised in that said protein is present in a form having a salt content of less than 5% by weight based on the weight of said protein.

2. An implant as claimed in claim 1 wherein said protein is selected from recombinant proteins having bovine growth hormone or porcine growth hormone activity.

3. An implant as claimed in claim 1 or 2 having a compressed matrix comprising said protein mixed with a biocompatible water-insoluble polymer.

4. An implant as claimed in claim 3 wherein said polymer is ethyl cellulose.

5. An implant as claimed in claim 4 wherein said matrix contains 30% by weight of said protein, 30% by weight of ethyl cellulose and 40% by weight of sucrose.

6. An implant as claimed in any one of claims 3 to 5 wherein said matrix is of a generally cylindrical shape within a tube of a microporous polymer film sealed at each end with non-porous polymer film.

7. An implant as claimed in claim 6 wherein said matrix is within a tube of microporous polyethylene film sealed at each end with non-porous polyethylene film.

8. A method of preparing an implant which comprises :
a) subjecting a solution of a recombinant protein having the activity of an animal growth hormone, which has been recovered from transformant cells in a fermentation medium, to one or more dialysis steps at a pH no lower than physiological pH so as to obtain an aqueous solution of said protein having a salt content which is reduced relative to that of the starting solution;
b) lyophilizing an aqueous solution of said protein at a pH no lower than physiological pH to produce a lyophilisate which has a salt content of less than 5% by weight based on the weight of said recombinant protein; and
c) formulating the lyophilized protein thus obtained into said implant.

9. A method as claimed in claim 8 wherein in step (c) a compressible composition comprising the lyophilized protein mixed with a biocompatible water-insoluble polymer is formed and the said composition is compressed into a unitary dosage matrix.

10. A method as claimed in claim 9 wherein said polymer is ethyl cellulose.

11. A method as claimed in any one of claims 8 to 10 wherein said protein is selected from recombinant proteins having bovine growth hormone or porcine growth hormone activity.

12. A method as claimed in any one of claims 8 to 11 wherein one or more dialysis steps comprising dialyzing an aqueous buffer solution of said protein against a dialysis buffer at a pH no lower than physiological pH are preceded and/or followed by one or more ultrafiltration steps to concentrate the said protein.

13. A method as claimed in claim 12 wherein said protein has bovine growth hormone activity and is dialyzed against progressive dilutions of CB- buffer, the dialysis buffers having a pH of 9.6 to 10.0.

14. A method as claimed in claim 13 wherein said protein is dialyzed successively against 1x,0.20x, 0.05x and 0.01x dilutions of CB⁻buffer.

15. A method as claimed in claim 12 wherein said protein has bovine growth hormone activity and is dialyzed against a single dialysis buffer at pH 9.8, said dialysis buffer being a 0.01x or 0.02x dilution of CB⁻buffer.

16. A method as claimed in claim 12 wherein said protein has porcine growth hormone activity and is dialyzed against 2-5 mM Tris buffer having a pH of 7.4.

17. A method of promoting growth of an animal which comprises subcutaneous implantation of an implant as claimed in any one of claims 1 to 7.

## Claims (Claims for the following Contracting State(s): AT)

1. A method of preparing an implant, for subcutaneous administration to an animal of a growth promoting amount of a recombinant protein having growth hormone activity which comprises:
a) subjecting a solution of a recombinant protein having the activity of an animal growth hormone, which has been recovered from transformant cells in a fermentation medium, to one or more dialysis steps at a pH no lower than physiological pH so as to obtain an aqueous solution of said protein having a salt content which is reduced relative to that of the starting solution;
b) lyophilizing an aqueous solution of said protein at a pH no lower than physiological pH to produce a lyophilisate which has a salt content of less than 5% by weight based on the weight of said recombinant protein; and
c) formulating the lyophilized protein thus obtained into said implant.

2. A method as claimed in claim 1 wherein in step (c) a compressible composition comprising the lyophilized protein mixed with a biocompatible water-insoluble polymer is formed and the said composition is compressed into a unitary dosage matrix.

3. A method as claimed in claim 2 wherein said polymer is ethyl cellulose.

4. A method as claimed in any one of claims 1 to 3 wherein said protein is selected from recombinant proteins having bovine growth hormone or porcine growth hormone activity.

5. A method as claimed in any one of claims 1 to 4 wherein one or more dialysis steps comprising dialyzing an aqueous buffer solution of said protein against a dialysis buffer at a pH no lower than physiological pH are preceded and/or followed by one or more ultrafiltration steps to concentrate the said protein.

6. A method as claimed in claim 5 wherein said protein has bovine growth hormone activity and is dialyzed against progressive dilutions of CB⁻buffer, the dialysis buffers having a pH of 9.6 to 10.0.

7. A method as claimed in claim 6 wherein said protein is dialyzed successively against 1x0.20x, 0.05x and 0.01x dilutions of CB⁻buffer.

8. A method as claimed in claim 5 wherein said protein has bovine growth hormone activity and is dialyzed against a single dialysis buffer at pH 9.8, said dialysis buffer being a 0.01x or 0.02x dilution of CB⁻buffer.

9. A method as claimed in claim 5 wherein said protein has porcine growth hormone activity and is dialyzed against 2-5 mM Tris buffer having a pH of 7.4.

10. A method as claimed in claim 3 wherein in said implant the matrix contains 30% by weight of said protein, 30% by weight of ethyl cellulose and 40% by weight of sucrose.

11. A method as claimed in any one of claims 2, 3 or 10 wherein in said implant said matrix is of a generally cylindrical shape within a tube of a microporous polymer film sealed at each end with non-porous polymer film.

12. A method as claimed in claim 11 wherein in said implant said matrix is within a tube of microporous polyethylene film sealed at each end with non-porous polyethylene film.

13. A method of promoting growth of an animal which comprises subcutaneous implantation of an implant as defined in any one of claims 1 to 12.

## Patentansprüche (Patentansprüche für folgende(n) Vertragsstaat(en): BE, CH, DE, FR, GB, IT, LI, LU, NL, SE)

1. Implantat mit verzögerter Abgabe zur subkutanen Verabreichung einer wachstumsfördernden Menge eines rekombinanten Proteins mit Wachstumshormon-Aktivität an ein Tier, dadurch gekennzeichnet, daß das Protein in einer Form vorliegt, die einen Salzgehalt von weniger als 5 Gew.-%, bezogen auf das Gewicht des Proteins, aufweist.

2. Implantat nach Anspruch 1, worin das Protein ausgewählt ist unter rekombinanten Proteinen mit Rinder-Wachstumshormon- oder Schweine-Wachstumshormon-Aktivität.

3. Implantat nach Anspruch 1 oder 2 mit einer komprimierten Matrix, welche das Protein, vermischt mit einem biologisch verträglichen, wasserunlöslichen Polymer, umfaßt.

4. Implantat nach Anspruch 3, worin das Polymer Ethylcellulose ist.

5. Implantat nach Anspruch 4, worin die Matrix 30 Gew.-% Protein, 30 Gew.-% Ethylcellulose und 40 Gew.-% Succrose enthält.

6. Implantat nach einem der Ansprüche 3 bis 5, worin die Matrix eine im allgemeinen zylindrische Form in einer Röhre aus einem mikroporösen Polymerfilm aufweist, die an jedem Ende mit einem nicht-porösen Polymerfilm verschlossen ist.

7. Implantat nach Anspruch 6, worin sich die Matrix in einer Röhre aus einem mikroporösen Polyethylenfilm befindet, die an jedem Ende mit einem nicht-porösen Polyethylenfilm verschlossen ist.

8. Verfahren zur Herstellung eines Implantates, wobei man:
a) eine Lösung eines rekombinanten Proteins mit der Aktivität eines tierischen Wachstumshormons, das aus transformierten Zellen in einem Fermentationsmedium isoliert wurde, einem oder mehreren Dialyseschritten bei einem pH unterzieht, der nicht niedriger ist als der physiologische PH, so daß man eine wäßrige Lösung des Proteins erhält, die einen Salzgehalt aufweist, der, bezogen auf die Ausgangslösung, relativ verringert ist;
b) eine wäßrige Lösung des Proteins bei einem pH, der nicht niedriger ist als der physiologische pH, lyophilisiert, um ein Lyophilisat herzustellen, das einen Salzgehalt aufweist, der geringer als 5 Gew.-%, bezogen auf das Gewicht des rekombinanten Proteins ist; und
c) das auf diese Weise erhaltene lyophilisierte Protein zu dem Implantat formuliert.

9. Verfahren nach Anspruch 8, worin in Schritt c) eine komprimierbare Zusammensetzung ausgebildet wird, welche das lyophilisierte Protein im Gemisch mit einem biologisch verträglichen, wasserunlöslichen Polymer umfaßt, und die Zusammensetzung zu einer einheitlichen Dosierungsmatrix komprimiert wird.

10. Verfahren nach Anspruch 9, worin das Polymer Ethylcellulose ist.

11. Verfahren nach einem der Ansprüche 8 bis 10, worin das Protein ausgewählt ist unter rekombinanten Proteinen mit Rinder-Wachstumshormon- oder Schweine-Wachstumshormon-Aktivität.

12. Verfahren nach einem der Ansprüche 8 bis 11, worin ein oder mehrere Dialyseschritte durchgeführt werden, umfassend die Dialyse einer wäßrigen, gepufferten Lösung des Proteins gegen einen Dialysepuffer bei einem pH, der nicht niedriger ist als der physiologische pH-Wert, und/oder gefolgt durch einen oder mehrere Ultrafiltrationsschritte, um das Protein zu konzentrieren.

13. Verfahren nach Anspruch 12, worin das Protein Rinder-Wachstumshormon-Aktivität aufweist und gegen zunehmende Verdünnungen von CB⁻-Puffer dialysiert wird, wobei die Dialysepuffer einen pH-Wert von 9,6 bis 10,0 aufweisen.

14. Verfahren nach Anspruch 13, worin das Protein nacheinander gegen 1x, 0,20x, 0,05x und 0,01x-Verdünnungen von CB⁻-Puffern dialysiert wird.

15. Verfahren nach Anspruch 12, worin das Protein Rinder-Wachstumshormon-Aktivität aufweist und einmal gegen Dialysepuffer bei pH 9,8 dialysiert wird, wobei der Dialysepuffer eine 0,01x oder 0,02x-Verdünnung von CB⁻-Puffer ist.

16. Verfahren nach Anspruch 12, worin das Protein Schweine-Wachstumshormon-Aktivität aufweist und dialysiert wird gegen 2-5 mM Tris-Puffer mit einem pH von 7,4.

17. Verfahren zur Förderung des Wachstums eines Tiers, wobei man ein Implantat gemäß einem der Ansprüche 1 bis 7 subkutan implantiert.

## Patentansprüche (Patentansprüche für folgende(n) Vertragsstaat(en): AT)

1. Verfahren zur Herstellung eines Implantates zur subkutanen Verabreichung einer wachstumsfördernden Menge eines rekombinanten Proteins mit Wachstumshormon-Aktivität an ein Tier, wobei man:
a) eine Lösung eines rekombinanten Proteins mit der Aktivität eines tierischen Wachstumshormons, das aus transformierten Zellen in einem Fermentationsmedium isoliert wurde, einem oder mehreren Dialyseschritten bei einem pH unterzieht, der nicht niedriger ist als der physiologische pH, so daß man eine wäßrige Lösung des Proteins erhält, die einen Salzgehalt aufweist, der, bezogen auf die Ausgangslösung, relativ verringert ist;
b) eine wäßrige Lösung des Proteins bei einem pH, der nicht niedriger ist als der physiologische pH, lyophilisiert, um ein Lyophilisat herzustellen, das einen Salzgehalt aufweist, der geringer als 5 Gew.-%, bezogen auf das Gewicht des rekombinanten Proteins ist; und
c) das auf diese Weise erhaltene lyophilisierte Protein zu dem Implantat formuliert.

2. Verfahren nach Anspruch 1, worin in Schritt c) eine komprimierbare Zusammensetzung ausgebildet wird, welche das lyophilisierte Protein im Gemisch mit einem biologisch verträglichen, wasserunlöslichen Polymer umfaßt, und die Zusammensetzung zu einer einheitlichen Dosierungsmatrix komprimiert wird.

3. Verfahren nach Anspruch 2, worin das Polymer Ethylcellulose ist.

4. Verfahren nach einem der Ansprüche 1 bis 3, worin das Protein ausgewählt ist unter rekombinanten Proteinen mit Rinder-Wachstumshormon- oder Schweine-Wachstumshormon-Aktivität.

5. Verfahren nach einem der Ansprüche 1 bis 4, worin ein oder mehrere Dialyseschritte durchgeführt werden, umfassend die Dialyse einer wäßrigen, gepufferten Lösung des Proteins gegen einen Dialysepuffer bei einem pH, der nicht niedriger ist als der physiologische pH-Wert, und/oder gefolgt durch einen oder mehrere Ultrafiltrationsschritte, um das Protein zu konzentrieren.

6. Verfahren nach Anspruch 5, worin das Protein Rinder-Wachstumshormon-Aktivität aufweist und gegen zunehmende Verdünnungen von CB⁻-Puffer dialysiert wird, wobei die Dialysepuffer einen pH-Wert von 9,6 bis 10,0 aufweisen.

7. Verfahren nach Anspruch 6, worin das Protein nacheinander gegen 1x, 0,20x, 0,05x und 0,01x-Verdünnungen von CB⁻-Puffern dialysiert wird.

8. Verfahren nach Anspruch 5, worin das Protein Rinder-Wachstumshormon-Aktivität aufweist und einmal gegen Dialysepuffer bei pH 9,8 dialysiert wird, wobei der Dialysepuffer eine 0,01x oder 0,02x-Verdünnung von CB⁻-Puffer ist.

9. Verfahren nach Anspruch 5, worin das Protein Schweine-Wachstumshormon-Aktivität aufweist und dialysiert wird gegen 2-5 mM Tris-Puffer mit einem pH von 7,4.

10. Verfahren nach Anspruch 3, worin in dem Implantat die Matrix 30 Gew.-% des Proteins, 30 Gew.-% Ethylcellulose und 40 Gew.-% Succrose enthält.

11. Verfahren nach einem der Ansprüche 2, 3 oder 10, worin in dem Implantat die Matrix eine im allgemeinen zylindrische Form in einer Röhre aus einem mikroporösen Polymerfilm aufweist, die an jedem Ende mit einem nicht-porösen Polymerfilm verschlossen ist.

12. Verfahren nach Anspruch 11, worin in dem Implantat die Matrix in einer Röhre aus mikroporösem Polyethylenfilm vorliegt, welche an jedem Ende mit einem nicht-porösen Polyethylenfilm verschlossen ist.

13. Verfahren zur Förderung des Wachstums eines Tiers, wobei man ein Implantat gemäß einem der Ansprüche 1 bis 12 subkutan implantiert.

## Revendications (Revendications pour l'(les) Etat(s) contractant(s) suivant(s): BE, CH, DE, FR, GB, IT, LI, LU, NL, SE)

1. Implant à libération réglée de la substance active, destiné à l'administration sous-cutanée à un animal d'une proportion favorisant la croissance d'une protéine recombinante possédant une activité d'hormone de croissance, caractérisé en ce que la protéine en question est présente sous une forme qui possède une teneur en sel inférieure à 5 % en poids, sur base du poids de la protéine en cause.

2. Implant suivant la revendication 1, caractérisé en ce que l'on choisit la protéine concernée parmi les protéines recombinantes possédant une activité d'hormone de croissance bovine et d'hormone de croissance porcine.

3. Implant suivant la revendication 1 ou 2, comportant une matrice comprimée comprenant ladite protéine mélangée à un polymère insoluble dans l'eau et biocompatible.

4. Implant suivant la revendication 3, caractérisé en ce que le polymère en question est l'éthylcellulose.

5. Implant suivant la revendication 4, caractérisé en ce que la matrice contient 30 % en poids de ladite protéine, 30 % en poids d'éthylcellulose et 40 % en poids de saccharose.

6. Implant suivant l'une quelconque des revendications 3 à 5, caractérisé en ce que la matrice a une forme cylindrique dans son ensemble dans un tube en un film de polymère microporeux hermétiquement fermé à chaque extrémité par un film de polymère non poreux.

7. Implant suivant la revendication 6, caractérisé en ce que la matrice se trouve dans un tube d'un film de polyéthylène microporeux hermétiquement fermé à chaque extrémité par un film de polyéthylène non poreux.

8. Procédé de préparation d'un implant, caractérisé en ce que :
a) on soumet une solution d'une protéine recombinante possédant l'activité d'une hormone de croissance pour les animaux, qui a été récupérée à partir de cellules transformantes dans un milieu de fermentation, à une ou plusieurs étapes de dialyse à un pH non inférieur au pH physiologique, de façon à obtenir une solution aqueuse de la protéine précitée possédant une teneur en sel qui est réduite par rapport à celle de la solution de départ,
b) on lyophilise une solution aqueuse de ladite protéine à un pH non inférieur au pH physioloqique, de manière à obtenir un lyophilisat ou produit lyophilisé qui possède une teneur en sel inférieure à 5 % en poids sur base du poids de ladite protéine recombinante et
c) on introduit la protéine lyophilisée ainsi obtenue dans l'implant concerné.

9. Procédé suivant la revendication 8, caractérisé en ce qu'au cours de l'étape (c), on forme une composition compressible comprenant la protéine lyophilisée mélangée à un polymère insoluble dans l'eau et biocompatible et on comprime la composition ainsi obtenue en une matrice à dose unitaire.

10. Procédé suivant la revendication 9, caractérisé en ce que le polymère est l'éthylcellulose.

11. Procédé suivant l'une quelconque des revendications 8 à 10, caractérisé en ce que l'on choisit la protéine parmi les protéines recombinantes possédant une activité d'hormone de croissance bovine ou une activité d'hormone de croissance porcine.

12. Procédé suivant l'une quelconque des renvendications 8 à 11, caractérisé en ce qu'une ou plusieurs étapes de dialyse comprenant la dialyse d'une solution aqueuse tampon de ladite protéine vis-à-vis d'un tampon de dialyse à un pH non inférieur au pH physiologique sont précédées et/ou suivies d'une ou plusieurs étapes d'ultrafiltration destinées à concentrer ladite protéine.

13. Procédé suivant la revendication 12, caractérisé en ce que ladite protéine possède une activité d'hormone de croissance bovine et est dialysée vis-à-vis de dilutions progressives de tampons CB⁻, les tampons de dialyse possédant un pH de 9,6 à 10,0.

14. Procédé suivant la revendication 13, caractérisé en ce que la protéine est dialysée successivement vis-à-vis de dilutions 1x, 0,20x, 0,05x et 0,01x du tampon CB⁻.

15. Procédé suivant la revendication 12, caractérisé en ce que la protéine possède une activité d'hormone de croissance bovine et est dialysée vis-à-vis d'un tampon de dialyse unique à un pH de 9,8, ledit tampon de dialyse étant une dilution 0,01x ou 0,02x du tampon CB⁻.

16. Procédé suivant la revendication 12, caractérisé en ce que ladite protéine possède une activité d'hormone de croissance porcine et est dialysée vis-à-vis d'un tampon 2- 5mM Tris possédant un pH de 7,4.

17. Procédé pour favoriser la croissance d'un animal, caractérisé en ce qu'il comprend l'implantation sous-cutanée d'un implant suivant l'une quelconque des revendications 1 à 7.

## Revendications (Revendications pour l'(les) Etat(s) contractant(s) suivant(s): AT)

1. Procédé de préparation d'un implant destiné à l'administration sous-cutanée à un animal d'une proportion favorisant la croissance d'une protéine recombinante possédant une activité d'hormone de croissance, caractérisé en ce que la protéine en question est présente sous une forme qui possède une teneur en sel inférieure à 5 % en poids, sur base du poids de la protéine en cause.

2. Procédé suivant la revendication 1 caractérisé en ce qu'au cours de l'étape (c), on forme une composition compressible comprenant la protéine lyophilisée mélangée à un polymère insoluble dans l'eau et biocompatible et on comprime la composition ainsi obtenue en une matrice à dose unitaire.

3. Procédé suivant la revendication 2, caractérisé en ce que le polymère est l'éthylcellulose.

4. Procédé suivant l'une quelconque des revendications 1 à 3, caractérisé en ce que l'on choisit la protéine parmi les protéines recombinantes possédant une activité d'hormone de croissance bovine ou une activité d'hormone de croissance porcine.

5. Procédé suivant l'une quelconque des revendications 1 à 4, caractérisé en ce qu'une ou plusieurs étapes de dialyse comprenant la dialyse d'une solution aqueuse tampon de ladite protéine vis-à-vis d'un tampon de dialyse à un pH non inférieur au pH physiologique sont précédées et/ou suivies d'une ou plusieurs étapes d'ultrafiltration destinées à concentrer ladite protéine.

6. Procédé suivant la revendication 5, caractérisé en ce que ladite protéine possède une activité d'hormone de croissance bovine et est dialysée vis-à-vis de dilutions progressives de tampons CB⁻, les tampons de dialyse possédant un pH de 9,6 à 10,0.

7. Procédé suivant la revendication 6, caractérisé en ce que la protéine est dialysée successivement vis-à-vis de dilutions 1x, 0,20x, 0,05x et 0,01x du tampon CB⁻.

8. Procédé suivant la revendication 5, caractérisé en ce que la protéine possède une activité d'hormone de croissance bovine et est dialysée vis-à-vis d'un tampon de dialyse unique à un pH de 9,8, ledit tampon de dialyse étant une dilution 0,01x ou 0,02x du tampon CB⁻.

9. Procédé suivant la revendication 5, caractérisé en ce que ladite protéine possède une activité d'hormone de croissance porcine et est dialysée vis-à-vis d'un tampon 2- 5mM Tris possédant un pH de 7,4.

10. Procédé suivant la revendication 3, caractérisé en ce que dans ledit implant, la matrice contient 30 % en poids de ladite protéine, 30 % en poids d'éthylcellulose et 40 % en poids de saccharose.

11. Procédé suivant l'une quelconque des revendications 2, 3 et 10, caractérisé en ce que dans ledit implant, la matrice possède une forme cylindrique dans son ensemble dans un tube en un film de polymère microporeux, hermétiquement fermé à chaque extrémité par un film de polymère non poreux.

12. Procédé suivant la revendication 11, caractérisé en ce que dans ledit implant, ladite matrice se trouve dans un film de polyéthylène microporeux hermétiquement fermé à chaque extrémité par un film de polyéthylène non poreux.

13. Procédé pour favoriser la croissance d'un animal, caractérisé en ce qu'il comprend l'implantation sous-cutanée d'un implant tel que défini dans l'une quelconque des revendications 1 à 12.
